# EUROPEAN PATENT APPLICATION

(11) **EP 4 818 678 A1**
(43) Date of publication of application: **07.10.2026**
(21) Application number: 24897400.8
(22) Date of filing: 20.11.2024
(51) Int. Cl.: G01N 21/78, A61B 5/00, G01N 33/483, G16H 30/40, G16H 50/30

(54) **BIOLOGICAL INFORMATION INFERENCE DEVICE, BIOLOGICAL INFORMATION INFERENCE METHOD, AND PROGRAM**

(30) Priority: 28.11.2023 JP 2023200410; 28.11.2023 JP 2023200846
(71) Applicant: Toppan Holdings Inc., Tokyo 110-0016 (JP)
(72) Inventor: MIYOSHI Yuki, Tokyo 110-0016 (JP); CHIBA Takato, Tokyo 110-0016 (JP)
(74) Representative: TBK
(86) International application number: PCT/JP2024/041090
(87) International publication number: WO 2025/115714

(57) **Abstract**

One mode of the present disclosure is a biological information estimation apparatus provided with an image acquisition unit that acquires an image capturing a subject; a preprocessing unit that performs a predetermined preprocessing on the captured image; an extraction unit that extracts color information of the subject from the captured image preprocessed by the preprocessing unit; an estimation unit that estimates a biological information value based on the color information and subject information indicating information about the subject, the biological information value being a value indicating biological information; and an output unit that outputs the biological information value. The preprocessing performed in the preprocessing unit includes a color correction process. The color correction process corrects a color of the subject included in the captured image to approximate an actual color of the subject.

## Description

### [Technical Field]

The present invention relates to a biological information estimation apparatus, a biological information estimation method, and a program.

### [Background Art]

It is known that technology exists for estimating health status using images of a human body. For example, Patent Document 1 describes a health status estimation system that estimates the health status of a person being checked, by analyzing images of their nails. The health status estimation system acquires an image including the nails of the person being checked, extracts features from the image, and estimates the health status of the person based on machine learning which uses the extracted features.

It is also known that technology exists for performing tests such as urine tests while a person being checked is at home.

For example, Patent Document 2 describes a urine testing device that captures images of urine test reagents using a digital camera. The urine testing device has a through hole for inserting the lens barrel of a digital camera into the opening of a container having a urine test reagent provided on its inner bottom surface. This allows the digital camera to be fixed to the urine testing device to image a reference standard display and a urine test reagent after a color reaction. After capturing the images, the captured image are displayed on a display monitor, thus enabling comparison and evaluation between the color of the reference standard display and the color of the urine test reagent.

Patent Document 3 is provided to have an imaging device. This imaging device captures both color change information and color change reference information. The color change information is multiple types of color change information exhibited by a color change portion that can change to multiple colors upon contact with urine. Furthermore, the color change reference information is multiple sets of reference data indicating the correspondence relationship with measured values of living body information. According to Patent Document 3, the color change information captured by the imaging device is compared with the color change reference information, and the color change reference information corresponding to the captured color change information is identified.

### [Citation List]

### [Patent Literature]

PTL1: JP 2021-125130 A
PTL2: JP 2009-229212 A
PTL3: JP 2015-187562 A

### [Summary of the Invention]

### [Problem to be Solved by the Invention]

The technology described in Patent Document 1 uses images to estimate health status based on images of the human body. However, the camera device used to capture images and the surrounding environment in which images are captured vary for each person being checked. Differences in the camera device and surrounding environment result in variations in the color information of the images, thus making it difficult to estimate health status with high accuracy.

The inspection described in Patent Document 2 uses images captured by an imaging device. However, depending on the model and performance of the digital camera, it may not be possible to obtain accurate color images, which can prevent the inspection from being performed accurately.

In the inspection described in Patent Document 3, it is necessary to simultaneously capture both the color change information and the color change reference information, requiring the preparation of the color change reference information for each shot. Furthermore, in this inspection, the color change information and the color change reference information may also vary depending on the surrounding environment. Hence, similar to the situation described in Patent Document 1, it is susceptible to the influence of the digital camera model and performance, and it may not be possible to obtain accurate color images.

Thus, this disclosure is provided in light of the foregoing various circumstances and aims to provide a biological information estimation apparatus, a biological information estimation method, and a program capable of acquiring color information from captured images and estimating biological information with a higher accuracy.

### [Solution to Problem]

The present disclosure is made to solve the foregoing problems. One aspect of the present disclosure is provided as a biological information estimation apparatus including: an image acquisition unit that acquires an image capturing a subject; a preprocessing unit that performs a predetermined preprocessing on the captured image; an extraction unit that extracts color information of the subject from the captured image preprocessed by the preprocessing unit; an estimation unit that estimates a biological information value based on the color information and subject information indicating information about the subject, the biological information value being a value indicating biological information; and an output unit that outputs the biological information value.

Another aspect of the present disclosure is provided as a biological information estimation method, which is performed by a computer, comprising steps of: acquiring an image capturing a subject; performing a predetermined preprocessing on the captured image; extracting color information of the subject from the captured image preprocessed by the preprocessing unit; estimating a biological information value based on the color information and subject information indicating information about the subject, the biological information value being a value indicating biological information; and outputting the biological information value.

Still another aspect of the present disclosure is provided as a biological information estimation program which enables a computer to perform steps of: acquiring an image capturing a subject; performing a predetermined preprocessing on the captured image; extracting color information of the subject from the captured image preprocessed by the preprocessing unit; estimating a biological information value based on the color information and subject information indicating information about the subject, the biological information value being a value indicating biological information; and outputting the biological information value.

### [Advantageous Effect of Invention]

According to one aspect of the present invention, biological information can be estimated with higher accuracy by acquiring color information from captured images.

### [Brief Description of Drawings]

[Fig. 1] Fig. 1 is a block diagram exemplifying the configuration of a biological information estimation system provided in a first embodiment.
[Fig. 2] Fig. 2 is a block diagram exemplifying the configuration of a biological information estimation system provided in a second embodiment.
[Fig. 3] Fig. 3 is a block diagram exemplifying processing procedures performed in the biological information estimation system in the second embodiment.
[Fig. 4] Fig. 4 is a block diagram exemplifying a color information acquisition device provided in the second embodiment.
[Fig. 5] Fig. 5 is a block diagram exemplifying the biological information estimation system provided in the second embodiment.
[Fig. 6] Fig. 6 is a block diagram exemplifying another biological information estimation system provided in the second embodiment.
[Fig. 7] Fig. 7 is a block diagram exemplifying the configuration of an examination system employed in a third embodiment.
[Fig. 8] Fig. 8 is a sequence diagram exemplifying processing procedures performed by the examination system in the third embodiment.
[Fig. 9] Fig. 9 is an illustration exemplifying screens displayed by a terminal employed in the third embodiment.
[Fig. 10] Fig. 10 is a block diagram exemplifying an image-color correction device provided in the third embodiment.
[Fig. 11] Fig. 11 is a block diagram exemplifying an examination system employed by the third embodiment.

### [Description of the Embodiments]

In the following, a biological information estimation apparatus, a biological information estimation method, and a program, to which the present invention is applied, will now be described with reference to the accompanying drawings.

### (First Embodiment)

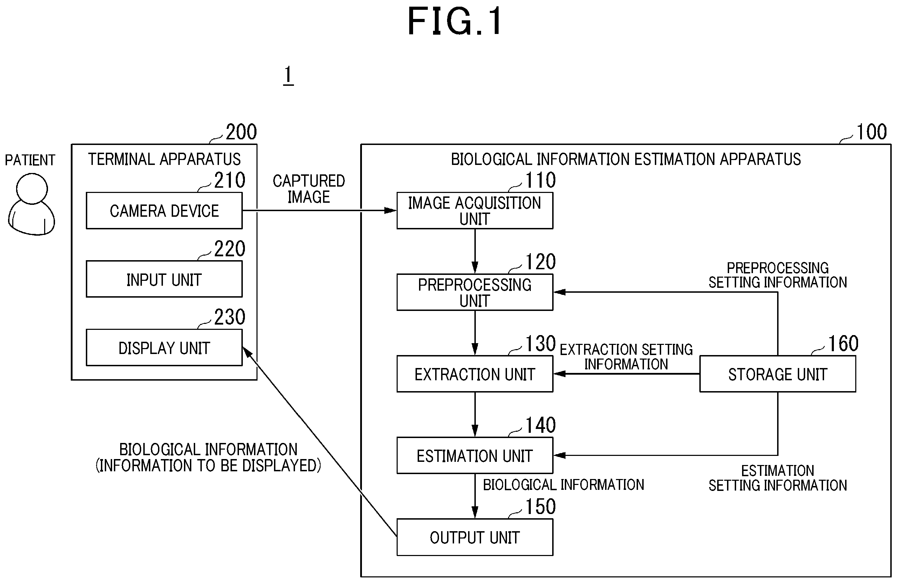

Fig. 1 is a block diagram showing an example configuration of a biological information estimation system 1 employed in the first embodiment. The biological information estimation system 1 is provided as a system which transmits captured images to a biological information estimation apparatus 100, which is disposed remotely from a subject, and enables the biological information estimation apparatus 100 to acquire color information from the captured images and then estimate, from the acquired color information, biological information with higher accuracy.

Subjects in the first embodiment include body parts of persons being checked and reagents which are reacted with user's bodily fluids. The persons being checked are users of a terminal device 200, patients, etc. Biological information refers to various types of numerical values relating to the physiology of the person being checked. The biological information includes values measured directly from a living body, such as body temperature, blood pressure, oxygen saturation, and/or pulse rate, or test values obtained by reacting bodily fluids with reagents or similar agents.

The biological information estimation system 1 may be provided with, for example, the biological information estimation apparatus 100 and the terminal device 200 equipped with a camera device 210. The biological information estimation apparatus 100 and the terminal device 200 are connected, for example, via a communication network. The biological information estimation apparatus 100 and terminal device 200 possess a communication interface, such as a Network Interface Card (NIC) or wireless communication module, for connecting to networks such as the Internet. The communication network may include, for example, general-purpose networks such as the Internet, and private networks such as Local 5G or Wi-Fi (registered trademark).

The terminal device 200 is an information processing device such as a smartphone or personal computer used by a user. The terminal device 200 is provided with a camera device 210, an input unit 220, and a display unit 230.

The camera device 210 performs operations based on commands received by the input unit 220, which include generating an image of a subject.

The input unit 220 is provided as a device that accepts user commands, such as those from touch sensors built into the display unit 230. The display unit 230 is provided as a device that displays various information, and is composed of, for example, an organic EL display.

The terminal device 200 works to execute application software, so that the terminal device 200 accepts user input, transmits captured images generated by the camera device 210 to the biological information estimating apparatus 100, and displays biological information (test results) transmitted from the biological information estimating apparatus 100.

It should be noted that, in the present embodiment, the terminal device 200 and the biological information estimating apparatus 100 are provided as mutually separate units, however, it is not limited to such a separate configuration. By way of example, the functions performed by the biological information estimating apparatus 100 may be incorporated within the terminal device 200.

The camera device 210 may be a camera built into a smartphone, a single-lens reflex camera, a webcam, or the like. The terminal device 200 may be configured to transmit other information, such as metadata indicating camera-related information, including the model of a camera provided in the terminal device 200, together with the captured image.

The captured image may be still images or moving images. The captured image may be multiple images generated by time-lapse photography.

The captured image may be an image capturing a part of the patient. Examples of such parts include the nails, the back of the hand, the face, the lips, the conjunctiva, the eyes, the eyelids, and the tongue. The image being obtained may be an image capturing multiple parts of the patient.

The captured image may be a still image of a reagent, or may be a moving image of a reagent. The captured image may include multiple images generated by time-lapse photography at intervals of seconds, for example to observe the reaction of reagents. The captured image may include multiple images captured by varying the subject distance, flash presence, angle, etc.

The captured image may be an image that includes both the patient and reference objects such as color charts or white plates for color correction. The captured image may include both an image containing the patient and an image capturing the reference objects.

The captured image may include depth information measured using a depth sensor or other sensing devices.

The biological information estimation apparatus 100 is provided as an information processing device that communicates with other devices and performs various processes. The biological information estimation apparatus 100 is provided with, by way of example, an image acquisition unit 110, a preprocessing unit 120, an extraction unit 130, an estimation unit 140, an output unit 150, and a storage unit 160. The image acquisition unit 110, preprocessing unit 120, extraction unit 130, estimation unit 140, and output unit 150 are functional units functionally realized by a processor, such as a CPU (Central Processing Unit), executing biological information estimation programs stored in a program memory.

The image acquisition unit 110 is configured to acquire captured images of a subject being examined.

The preprocessing unit 120 is configured to perform predetermined preprocessing on the captured image. The preprocessing unit 120 may perform the preprocessing by referring to the preprocessing setting information stored in the storage unit 160. In the present embodiment, the preprocessing setting information is provided as, for example, information which identifies processing rules such as preprocessing procedures and calculation formulas, programs, and machine learning models.

The preprocessing unit 120 may also perform color correction processing to adjust the colors of the subject which are depicted in the captured images to approximate the actual colors of the subject. The preprocessing unit 120 is also configured to correct the image to achieve the 'correct colors' of the subject in the captured images. The correct colors refer to colors that are correct in terms of colorimetric values (defined color values measured by a colorimeter).

The preprocessing unit 120 may additionally perform image processing useful for estimating biological information values, such as illuminance unevenness correction (shading correction), shadow correction, sharpness correction, and/or contrast correction. In addition, the preprocessing unit 120 may additionally perform processing such as enlarging, reducing, and/or shape adjustment of the captured images, and/or and object extraction from the captured images, provided it is useful for estimating the biological information values.

The extraction unit 130 is configured to extract subject's color information from the captured images preprocessed by the preprocessing unit 120. The extraction unit 130 may perform the extraction of color information by referring to extraction setting information stored in the storage unit 160. The extraction setting information is provided as information for performing the color information extraction process performed by the extraction unit 130, such as information indicating a body portion to be extracted, or information indicating a pre-trained spectral reflectance estimation model. Consequently, the extraction unit 130 is able to extract the color information corresponding to each item of biological information, even when, by way of example, estimating multiple pieces of the biological information.

The color information refers to data such as colorimetric values indicating the patient's body color for estimating biological information. The color information may be RGB values reproduced in a specific color space, such as RGB values or sRGB values with white balance correction applied. The color information may be information, such as spectral reflectance, XYZ values in the XYZ color space, L*a*b* values in the L*a*b* color space, erythema index, melanin index, absorbance, spectral data at specific wavelengths, representative values for specific regions of the subject, or time-series information showing temporal changes in images, such as video data with pixel-by-pixel values or images from interval photography. In addition, the color information may be provided so as to include two or more of these types of information. The color information may be a single value, such as an average value for the extracted body portion, or it may be image data (a two-dimensional distribution of color information) for a small region containing the extracted body portion.

The estimation unit 140 is configured to estimate a biological information value, which is a value indicating biological information based on the color information and the subject information. The subject information is provided as information indicating information about a subject which has been imaged (captured). Specifically, the subject information is given as information indicating a patient's body portion and information indicating a reagent. The estimation unit 140 may estimate the biological information using methods such as multiple regression analysis, polynomial approximation, or machine learning.

The estimation unit 140 may also perform estimation of the biological information by referring to the estimation setting information stored in the storage unit 160. The estimation setting information is given as, for example, information identifying processing rules such as estimation processing procedures and calculation formulas, programs, or machine learning models.

The output unit 150 is configured to output the biological information values. The output unit 150 may additionally transmit display data for displaying the biological information values on terminal device 200. Alternatively, the output unit 150 may be configured to output, for example, the biological information to a device storing the biological information, or output the biological information to an examination apparatus performing various examinations using the biological information. Moreover, the output unit 150 may output multiple pieces of the biological information at the same time.

The storage unit 160 is configured to store the preprocessing setting information, extraction setting information, and estimation setting information. The preprocessing setting information is, for example, information for correcting the color information without using a color chart. For example, this preprocessing setting information includes camera spectral sensitivity and camera tone characteristics for each camera model; a pre-trained spectral reflectance estimation model specific to each subject or a generic pre-trained spectral reflectance estimation model; the illumination spectral distribution; and parameters required to estimate the illumination color from the captured images. The preprocessing setting information is, for example, information indicating the measured color values for each patch on the color chart when the preprocessing unit 120 performs the color correction using the color chart.

The storage unit 160 may include the following information as the preprocessing setting information. Namely, such preprocessing setting information includes: white balance information, such as a pre-captured white paper image used for color unevenness (shading) correction; black balance information, such as a pre-captured black paper image used for shadow correction; sharpness when multiple biological information estimation targets exist; and/or parameters for optimizing each subject for contrast enhancement processing.

The storage unit 160 stores estimation setting information. The estimation setting information is provided as conversion information for estimating the biological information when the subject is a reagent. This conversion information is necessary as data indicating a correspondence table between the color information of a captured image for each reagent and the actual color of the reagent. In addition, the estimation setting information is also provided as conversion information for estimating the biological information when the subject is a body portion or a blood component. This conversion information indicates, for example, a correspondence table between the color information of captured images for each nail or blood component and the actual color of the reagent.

The biological information estimation system 1 may be provided with a biological information determining unit that determines the biological information estimated by the estimation unit 140. The biological information determining unit may be provided as an input unit 220 that acquires information which determines the biological information in response to user operations. The biological information determining unit may also be an image acquisition unit 110 that acquires information which determines the biological information inputted in response to user operation. The preprocessing unit 120 performs color correction on the subject corresponding to the biological information determined by the biological information determining unit. The subject corresponding to the biological information is a subject suitable for the determined biological information. A subject suitable for the biological information is, for example, a patient's body portion for measuring blood pressure, or a urine test strip for measuring urine test values.

The biological information estimation system 1 may be provided with a subject determining unit for determining a subject. The subject determining unit may be an input unit 220 that acquires information which determines the subject in response to user operations. The subject determining unit may also be an image acquisition unit 110 that acquires information which determines the subject inputted in response to user operations. The preprocessing unit 120 performs color correction on the captured image corresponding to the subject determined by the subject determining unit. The extraction unit 130 estimates biological information values based on subject information corresponding to the subject determined by the subject determining unit.

The preprocessing unit 120 may be configured to switch conversion information which indicates the correspondence between the color information of the captured image and the colorimetric values of the subject, based on the type of subject determined by the subject determining unit. The conversion information is stored in the storage unit 160 as preprocessing setting information. The conversion information is provided as information for converting the color information of the captured image and includes information indicating the correspondence between the pixel values of the captured image and the actual color measurement values of the subject. The correspondence between the pixel values of the captured image and the actual color measurement values of the subject depends, for example, on the color characteristics of the subject. Therefore, the conversion information is prepared for each subject according to the subject's color characteristics. The subject's color characteristics show the trend of its spectral reflectance. The subject's color characteristics may be predicted using a pre-trained spectral reflectance estimation model obtained by learning data showing the subject's spectral reflectance.

The preprocessing unit 120 may estimate spectral reflectance by inputting the captured image into a machine learning model (pre-trained spectral reflectance estimation) trained using the color information of the subject-specific captured image and the actual subject's spectral reflectance. The preprocessing unit 120 may then perform color correction on the captured image based on the subject's colorimetric values derived from the estimated spectral reflectance. The pre-trained spectral reflectance estimation is a machine learning model trained using the color information of subject-specific captured images and the actual subject's color (spectral reflectance, true values). Using this pre-trained spectral reflectance estimation enables high-accuracy estimation of spectral reflectance. Furthermore, a general-purpose pre-trained spectral reflectance estimation model, which is trained using a dataset comprising general imaging data and color information (correct values) of imaging subjects without identifying specific subjects, may also be employed. A subject's color (colorimetric values) can then be obtained from the estimated spectral reflectance by applying the spectral distribution of the observation illumination and the color matching functions. The machine learning model for spectral reflectance estimation may be a neural network, a method based on statistical analysis such as principal component analysis, or a machine learning algorithm such as multiple regression analysis.

The preprocessing unit 120 may estimate relevant information concerning the imaging from the captured image and perform color correction based on the estimated relevant information. For example, the preprocessing unit 120 may estimate the illumination color (or light source color or spectral distribution) around the subject as relevant information and perform color correction based on the estimated illumination color. The preprocessing unit 120 may estimate the illumination color (or light source color and spectral distribution) around the subject by referencing parameters necessary for estimating the illumination color from the captured image. This estimation may be performed, for example, by determining the time of day at the imaging and whether the scene was outdoors or indoors, based on time information or latitude/longitude information attached to the captured image. The preprocessing unit 120 may, for example, refer to conversion information prepared according to the estimated illumination color and correct the color information of the captured image to bring it closer to the actual color of the subject.

The preprocessing unit 120 may perform the color correction based on camera model information included in the metadata of the captured image as relevant information. The camera model information includes camera color characteristic information, such as the spectral sensitivity and tonal characteristics of a camera, which correspond to the camera model. The preprocessing unit 120, for example, refers to conversion information prepared according to the camera model information extracted from the metadata and corrects the color information of the captured image to bring it closer to the actual color of the subject. Specifically, the preprocessing unit 120 can estimate spectral reflectance using the camera spectral sensitivity for each camera model stored in the storage unit 160, based on the camera model information, and switch the conversion information based on the estimated spectral reflectance.

The preprocessing unit 120 may estimate a spectral information image indicating a subject's spectral reflectance from the RGB image, which is an image captured of the subject, using a machine learning model. The preprocessing unit 120 inputs, into a machine learning model, the captured image, the spectral sensitivity of the camera device 210 that captured the image, and the spectral distribution of the light source in the environment where the image was captured, so that the machine learning mode enables estimation of the spectral information image. Consequently, the preprocessing unit 120 can apply preprocessing to the captured image whilst taking into account the spectral sensitivity of the camera device 210 and the light source in the environment where the image was captured.

The preprocessing unit 120 may perform the color correction based on registration information received according to the user's operations. The registration information is, for example, the illumination spectral distribution indicating the illumination color, and the preprocessing unit 120 may perform the color correction based on the illumination color. By way of example, the preprocessing unit 120 is configured to refer to the conversion information that transforms color information of the captured image corresponding to the illumination color to the actual subject's color, thereby correcting the color information of the captured image to bring it closer to the actual subject's color.

The registration information is terminal information, and the preprocessing unit 120 may perform the color correction based on camera model information corresponding to the terminal information. The preprocessing unit 120 references conversion information that transforms the color information of captured images, corresponding to registered camera model information, to the actual subject's color. Hence, the preprocessing unit 120 corrects the color information of captured images to approximate the actual subject's color.

The subject information may be set to indicate operations inducing temporal changes in the subject, and the estimation unit 140 may estimate biological information value based on changes in color information corresponding to temporal changes in the subject. Operations inducing temporal changes in the subject induce color changes or color enhancement in the body portion of the person being checked. The terminal device 200 prompts operations such that temporal changes are induced before or during the capture of the image and transmits the subject information to the biological information estimation apparatus 100.

The subject information may be information indicating actions such as compression. For example, the action information may be information indicating actions such as: before and after administration of a drug; before and after application of a drug; before and after application of electrical stimulation; before and after heating; before and after cooling; changes in room temperature and humidity; changes in oxygen concentration; before and after irradiation with light of a specific wavelength; and before and after administration of an allergen. The terminal device 200 may display a message to capture the patient's nail, display a message to compress the nail while the nail is being imaged, commence capturing moving images before and after compression, release the compression, and control the camera device 210 to continue imaging moving images after the compression is released. Thus, the biological information estimating apparatus 100 acquires the action of compressing a portion of the skin as subject information, and the terminal device 200 can transmit video of images when a portion of the skin was compressed, along with the subject information, to the biological information estimating apparatus 100. By way of example, the subject information may also be information measuring the time required for the nail color to return to its state prior to the imaging.

The estimation unit 140 estimates biological information based on the subject information and color information. Specifically, the estimation unit 140 transmits the subject information indicating the actions of pressing and releasing the fingernail to the terminal device 200.
The camera device 210 transmits images imaging the state from pressing the fingernail until its release to the biological information estimation apparatus 100. The extraction unit 130 acquires color information from the captured image, and the estimation unit 140 estimates biological information from the subject information and color information.

The subject information is biologically relevant information pertaining to the individual user. The estimation unit 140 may estimate a biological information value based on the color information and biologically relevant information. The biologically relevant information pertaining to the individual user includes information such as the user's gender, age, medical history, and biological information value from previous tests.

The subject information is either biologically relevant information pertaining to the individual user or environmental information. The estimation unit 140 may be configured to estimate biological information values based on the color information and biologically relevant information.

For example, in tests using reagents such as test strips, the biologically relevant information is provided as a correspondence table showing test values for each test item and the color of the corresponding reaction reagent. Test items include, for example, glucose test values and pH values. The color of the reaction reagent includes, for example, colorimetric values XYZ, L*a*b*, etc. For example, the correspondence table includes values such as colorimetric values "50, 4, -1" for test values of "-" for glucose, and colorimetric values "30, 2, -5" for test values of "+". For example, the correspondence table also includes values such as colorimetric values '20, 0, -2' for pH test values of '6.5', colorimetric values '40, 0, 2' for test values of '7.0', and colorimetric values '60, 2, -5' for test values 'of 7.5'.

The biologically relevant information is, for example, a correspondence table between biological information and the color of the body's portion corresponding to the biological information. The color of the reagent has, for example, colorimetric values XYZ, L*a*b*, etc. For example, if the body's portion is a fingernail and the biological information is 'hemoglobin', the correspondence table would be: colorimetric values '20, 0, -2' for test values of '6.0', colorimetric values '40, 0, 2' for test values of '8.0', and colorimetric values '60, 2, -5' for test values of '10.0'. For example, if the body's portion is a finger and the biological information is 'SpO2', the correspondence table includes values such as colorimetric values '20, 0, -2' for test values of'100', colorimetric values '40, 0, 2' for test values of '98', and colorimetric values '60, 2, -5' for test values of '96'.

The estimation unit 140 acquires colorimetric values serving as color information from the extraction unit 130 and can obtain test values corresponding to the colorimetric values. For example, if the color information acquired from the extraction unit 130 is the colorimetric values '50, 5, -1', the estimation unit 140 can classify and acquire a test value '-' corresponding to the colorimetric values '50, 4, -1', which are the closest in Euclidean distance, from the correspondence table. Furthermore, the estimation unit 140 may convert the test values into continuous values by performing regression processing. The classification or regression processing of test values performed by the estimation unit 140 may employ machine learning methods such as logistic regression analysis, multiple regression analysis, support vector machines, or neural networks.

The image acquisition unit 110 may be configured to acquire an image capturing the subject and a color chart image capturing the color chart, while the preprocessing unit 120 may be configured to use the color chart image for reference to the conversion information, which is for conversion of the color information included in an image of the subject imaged, and to correct the color information included in the captured image based on the conversion information. The color chart image has regions corresponding to multiple patches. The preprocessing unit 120 compares the color information included in the color chart image with the color information of the captured image, thereby determining a region within the color chart to be collated.

The biological information estimation apparatus 100 may be provided with a health-state estimation unit that estimates a health state based on the biological information value estimated by the estimation unit 140. For example, the health-state estimation unit may be implemented as one of the functions performed by the estimation unit 140. The health-state estimation unit estimates a user's health state based on the biological information value estimated by the estimation unit 140. The health-state estimation unit can be configured to estimate a disease name which indicates the health state. The health-state estimation unit may, for example, refer to the table information associating the biological information values stored in the storage unit 160 with the health states.

As described, according to the biological information estimation system 1 of the first embodiment, it is possible to apply predetermined preprocessing to an image which has been imaged, extract the subject's color information from the preprocessed image, and estimate a biological information value based on the color information and the subject information. According to this biological information estimation system 1, it is also possible to extract preprocessed color information and estimate biological information simply by capturing an image. Consequently, according to the biological information estimation system 1, the biological information can be estimated with higher accuracy. In addition, according to the biological information estimation system 1, the burden on the user who receives medical treatment or examination procedures can be reduced.

### (Second embodiment)

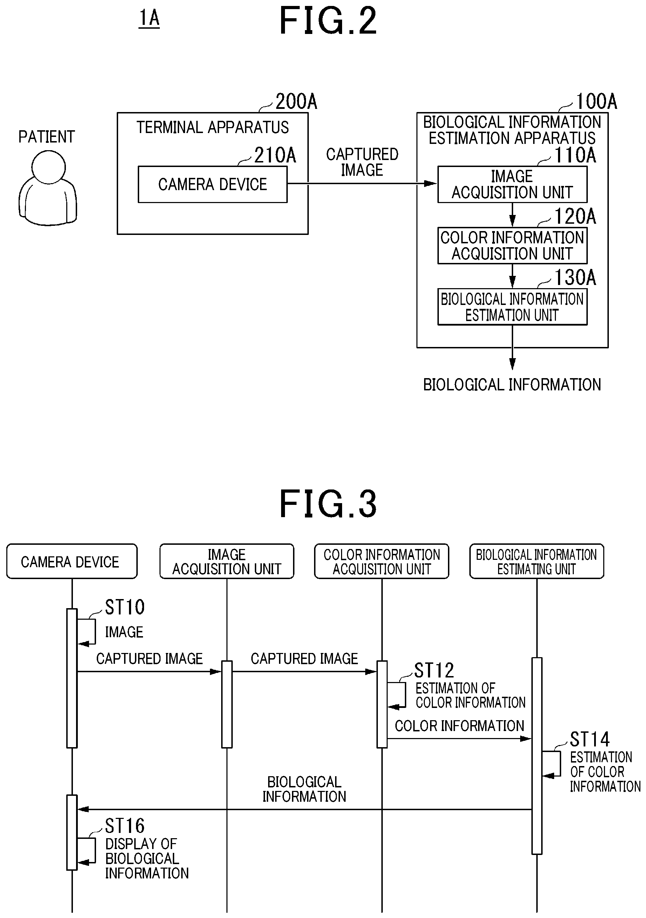

Fig. 2 is a block diagram exemplifying one configuration of a biological information estimation system 1A according to a second embodiment. It should be noted that the configurations described in the biological information estimation system in the foregoing embodiment may be applied to the biological information estimation system 1A which will now be described in the second embodiment.

The biological information estimation system 1A is configured to transmit captured images to a biological information estimation apparatus 100A disposed remotely from a living body, such as a patient. The biological information estimation apparatus 100A then acquires color information from the captured images to estimate biological information with higher accuracy. In the second embodiment, the subject for biological information estimation is a patient undergoing health examinations, but this is not limited to such cases, but it may be any human body or other living organism.

The biological information estimation system 1A is equipped with, for example, a biological information estimating apparatus 100A and a terminal device 200A equipped with a camera device 210A. The biological information estimation apparatus 100A and the terminal device 200A are connected, for example, via a communication network. The biological information estimation apparatus 100A and terminal device 200A possess communication interfaces, such as a NIC (Network Interface Card) or wireless communication module, for connecting to networks such as the Internet. The communication network may include, for example, general-purpose networks such as the Internet, and private networks such as local 5G or Wi-Fi (registered trademark).

The terminal device 200A is an information processing device, such as a smartphone or personal computer, used for examinations of patients. The terminal device 200A is equipped with a camera device 210A. The camera device 210A operates based on operations accepted by the terminal device 200A and generates captured images when imaging a patient. The terminal device 200A transmits the captured images to the biological information estimating apparatus 100. In the present embodiment, the terminal device 200A and the biological information estimation apparatus 100A are composed of mutually separate units, although this is not a limitation, and the functions performed by the biological information estimation apparatus 100A may be incorporated within the terminal device 200A.

The camera device 210A may be a camera built in a smartphone, a single-lens reflex camera, a webcam, or similar other imaging devices. The terminal device 200A may be configured to transmit information indicating the type of camera along with the captured images.

Images to be captured may be still images or moving images. The captured images may be provided as multiple images generated by interval photography.

The captured images may be images of a portion of the patient. Such portions of the patient include, for example, the nails, the back of the hand, the face, the lips, the conjunctiva, the eyes, the eyelids, and/or the tongue. The captured images may be images imaging multiple portions of the patient's body.

The captured images may be multiple images captured by varying a subject distance, flash usage, angle, etc.

The captured images may be images that include both the patient and reference objects such as color charts or white plates for color correction. The captured images may include both an image including the patient and an image capturing the reference objects.

The captured images may include depth information measured using a depth sensor or other similar components.

The biological information estimation apparatus 100A is an information processing device that communicates with other devices and performs various processes. For example, the biological information estimation apparatus 100A is provided with an image acquisition unit 110A, a color information acquisition device 120A, and a biological information estimation unit 130A. The image acquisition unit 110A, color information acquisition device 120A, and biological information estimation unit 130A are provided as functional units realized by a processor, such as a CPU (Central Processing Unit) which executes programs stored in a program memory.

The image acquisition unit 110A is configured to acquire images capturing a patient.

The color information acquisition device 120A is configured to correct the color information included in the captured images, and then acquire the corrected color information.

The color information can be provided as data indicating the patient's color for estimating patients' biological information. The color information may be RGB values reproduced in specific color spaces, such as RGB values or sRGB values, to which white balance correction is applied. The color information may also be spectral reflectance, XYZ values in the XYZ color space, L*a*b* values in the L*a*b* color space, erythema index, melanin index, absorbance, spectral data at specific wavelengths, representative values for specific regions of the subject, or time-series information showing temporal changes in images such as video data with pixel-by-pixel values or images from interval photography. Furthermore, the color information may include two or more pieces of such information. The color information may be a single value, such as the average value for the body portion being extracted, or it may be image data for a small region containing the body portion being extracted (a two-dimensional distribution of color information).

The biological information estimation unit 130A is configured to estimate the patient's biological information based on color information. The biological information estimation unit 130A estimates the biological information using methods such as multiple regression analysis, polynomial approximation, or machine learning. The biological information estimation apparatus 100A outputs the estimated biological information. By way of example, the biological information estimation apparatus 100A may output the estimated biological information to the terminal device 200A. Alternatively, the biological information estimating apparatus 100A may output the estimated biological information to a device for storing the biological information. As another alternative, the biological information estimating apparatus 100A may output the estimated biological information to an examination apparatus that performs various examinations using the received biological information. Moreover, the biological information estimation unit 130A can be configured such that the biological information estimation unit 130A estimates, at the same time, multiple pieces of biological information based on the color information.

Fig. 3 is a sequence diagram illustrating an example of the processing steps performed by the biological information estimation system 1A in the second embodiment.

First, the terminal device 200A captures an image of the patient using the camera device 210A (step ST10) and transmits the captured image to the biological information estimating apparatus 100A. The image acquisition unit 110A acquires the captured image and outputs it to the color information acquisition device 120A. The color information acquisition device 120A corrects color information included in the captured image so that this device 120A acquires the corrected color information (step ST12). The color information acquisition device 120A outputs the acquired color information to the biological information estimation unit 130A. The biological information estimation unit 130A estimates patient's biological information based on the acquired color information (Step ST14). The biological information estimation apparatus 100A transmits the biological information estimated by the biological information estimation unit 130A to the terminal device 200A. The terminal device 200A presents the biological information to the patient by displaying the biological information (Step ST16).

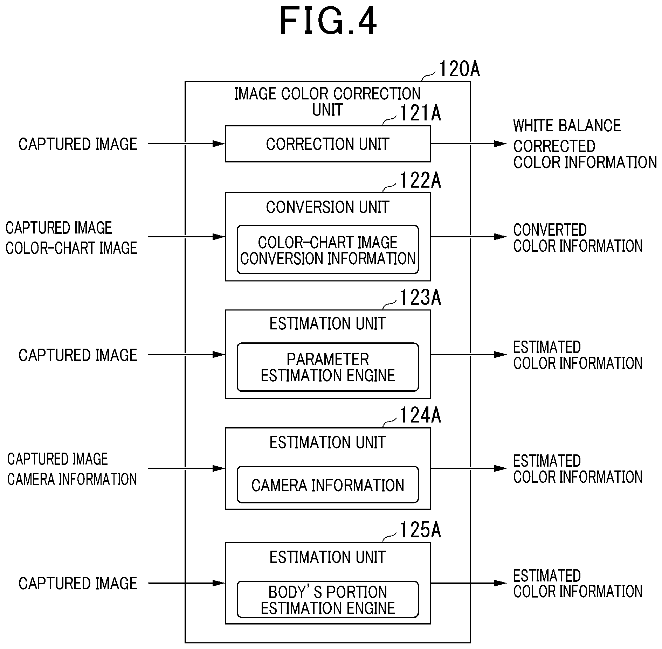

Fig. 4 is a diagram exemplifying the configurations of the color information acquisition device 120A in the second embodiment.

This color information acquisition device 120A can be provided to have a correction unit 121A, a conversion unit 122A, and at least one of estimation units 123A, 124A, and 125A. The correction unit 121A, the conversion unit 122A, and the estimation units 123A, 124A, and 125A may be, for example, functional units performed by a processor such as a CPU executing programs stored in a program memory.

The correction unit 121A estimates an illumination color of the space in which the patient was imaged based on the captured image and corrects the white balance of the RGB information included in the captured image. The correction unit 121A outputs the captured image, which includes color information with the corrected white balance, to the biological information estimation unit 130A.

The image acquisition unit 110A acquires a color chart image, which is an image of the color chart, and a captured image, which is an image of the patient. The conversion unit 122A generates conversion information by using the color chart image to convert the color information included in the captured image of the patient, and converts the color information included in the captured image based on the conversion information. The color chart is, for example, a plate-like object on which an array of color samples is arranged. The conversion unit 122A generates a color conversion matrix (serving as the conversion information) based on the relationship between the colors indicated by the color chart and the colors of the color chart included in the captured image. The conversion unit 122A generates the color conversion matrix using the color chart by performing, for example, multiple regression analysis. The conversion unit 122A can convert the captured image of the patient based on the conversion information, thereby converting the captured image to the colors indicated by the color chart. The conversion unit 122A outputs the converted captured image of the color information to the biological information estimation unit 130A.

The estimation unit 123A estimates color information based on output information from a machine learning engine that estimates parameters affecting the color of captured images when imaging a patient. The parameters affecting the color of captured images include, for example, spectral reflectance and/or absorbance. The machine learning engine serves as a parameter estimation engine trained using captured images and parameters (such as spectral reflectance and/or absorbance) serving as training data. The parameter estimation engine estimates parameters in response to the input of a captured image. The estimation unit 123A thus estimates the color information of the captured images using the parameters estimated by the parameter estimation engine.

The estimation unit 123A may generate other parameters, such as erythema index and melanin index, using the absorbance estimated by the parameter estimation engine. Alternatively, the estimation unit 123A may estimate the color information of the captured images using the other parameters generated using the parameters estimated by the parameter estimation engine.

The estimation unit 124A estimates correct color information based on the captured image acquired by the image acquisition unit 110A and color information, such as spectral sensitivity, of the camera that acquired the captured image. The terminal device 200A transmits the captured images along with information about the camera to the biological information estimating apparatus 100. The camera information can be provided as details related to color information, such as the type of camera device 210A and its specifications (e.g., sensitivity). The estimation unit 124A estimates the patient's color information based on the camera information included in the captured images.

The estimation unit 125A may estimate color information based on the body portion information acquired by the image acquisition unit 110A, or on the output information from a machine learning engine that recognizes a patient's body portion. The body portion information denotes information indicating body portions specified by the user, such as nails, the back of the hand, the face, or the conjunctivas. The machine learning engine that recognizes the patient's body portion can be provided as a body portion estimation engine trained using captured images and body portion information for identifying body portions as learning data. The body portion estimation engine estimates body portions in response to the input of the captured images. The estimation unit 125A estimates the color information of the captured image according to the body portion indicated by the body portion information, or the body portion estimated by the body portion estimation engine.

As described above, according to the biological information estimation system 1A according to the present embodiment, an image of a portion of the patient is acquired, the color information included in the captured image is corrected, and the patient's biological information is estimated based on the corrected color information. Hence, even for various captured images that vary depending on types of the camera device 210A used for imaging or the patient's surrounding environment, correcting the color information makes it possible to obtain color information from the captured images and estimate biological information with higher accuracy.

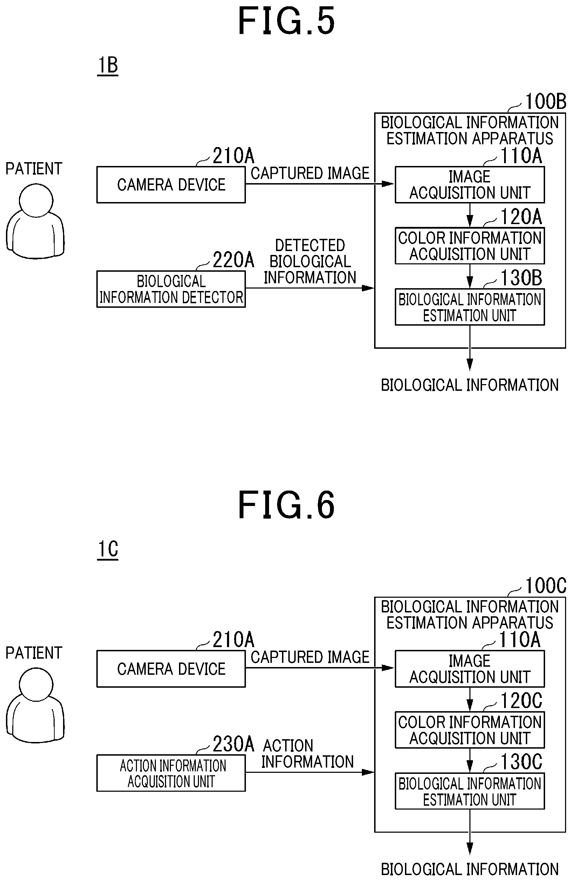

Fig. 5 is a block diagram illustrating an example of the biological information estimation system 1B provided in the present embodiment.

The biological information estimation system 1B is provided with a biological information detector 220A that acquires biological detection information detected from a patient. The biological information detector 220A may be a device, such as a smartwatch, that measures the patient's biological information, or may be a device that accepts operations from the patient or other persons and inputs biological information. By way of example, the biological information detector 220A can detect biological information that differs from the biological information estimated by the biological information estimation unit 130B. The biological information detected by the biological information detector 220A may be configured to provide biological information that assists the biological information estimation unit 130B in estimating the biological information. The biological information detected by the biological information detector 220A may be body temperature, oxygen saturation, pulse rate, blood pressure, electrocardiogram, etc., and may be an oxygen saturation measured by a pulse oximeter, or age, gender, blood type, height, weight, disease, etc., obtained via manual input. In addition, the biological information detector 220A may be communicably connected to a server device and may acquire blood test results (hemoglobin), urine test results, other test results (blood pressure, melanin, electrocardiogram, etc.), medical history, etc., from the server device. The biological information detector 220A may estimate biological information from the captured images obtained by the camera device 210A or by other means, through internal processing. Alternatively, the biological information detector 220A may estimate burns, injuries, melanin, etc., from the captured images.

The biological information estimation unit 130B estimates biological information based on the biological detection information acquired from the biological information detector 220A and the color information. Specifically, the biological information detector 220A transmits an image capturing the fingernail to the biological information estimating apparatus 100B. The biological information detector 220A transmits an oxygen saturation level acquired by a pulse oximeter or smartwatch to the biological information estimating apparatus 100B as biological detection information. The color information acquisition device 120A performs color correction on the nail image acquired by the image acquisition unit 110A to sRGB values and averages the nail's RGB values. The biological information estimation unit 130B estimates a hemoglobin value using polynomial approximation from both the color information corrected by the color information acquisition device 120A and the oxygen saturation transmitted from the biological information detector 220A.

As described above, according to the biological information estimating apparatus 100B described in the present embodiment, it is possible to estimate the patient's biological information based on the corrected color information and the biological detection information acquired by the biological information detector 220A, in addition to the images captured by imaging the patient.

Fig. 6 is a block diagram exemplifying the configuration of a biological information estimation system 1C according to the second embodiment.

The biological information estimation system 1C is provided with an action information acquisition unit 230A that acquires information about the patient's motions caused when imaging the imaging image. In this biological information estimation system 1C, the color information acquisition device 120C acquires the patient's color information based on the captured images and action information. The biological information estimation unit 130C estimates biological information based on the color information.

The action information on the patient, which is required in the imaging, is information indicating patient's motions that induce color changes or color enhancement appearing on the patient. The action information acquisition unit 230A may prompt the patient to perform operations to induce a change over time before or during the capture of the images. For example, the action information acquisition unit 230A may display a prompt message on the screen where the imaging device 210A captures the images and acquires action information indicating the motions corresponding to the image captured after displaying the message.

The action information may be information indicating actions such as compression. For example, the action information may be information indicating actions such as: before and after administration of a drug; before and after application of a drug; before and after application of electrical stimulation; before and after heating; before and after cooling; changes in room temperature and humidity; changes in oxygen concentration; before and after irradiation with light of a specific wavelength; and before and after administration of an allergen. In addition, the action information may represent the amount of operation performed when capturing videos.

Specifically, the action information acquisition unit 230A may control the camera device 210A such that a message to capture the patient's nail is displayed, a message to press the nail while it is being captured is displayed, capturing moving images before and after the pressing action is commenced, and even after the pressing action is released, capturing moving images is continued. Thereby, the action information acquisition unit 230A acquires the action information indicating the action of pressing a part of the skin, and the terminal device 200A can transmit both the captured moving images, which have been obtained when a part of the skin was pressed, and the action information to the biological information estimating apparatus 100C. Alternatively, the action information acquisition unit 230A may measure, as action information, the time required for the nail color to return to its state prior to the imaging, so that the action information acquisition unit 230A can acquire this time as one type of action information.

The biological information estimation unit 130C estimates biological information based on the action information, which is acquired from the action information acquisition unit 230A, and the color information. Specifically, the action information acquisition unit 230A transmits the action information indicating the actions of pressing and releasing the nail to the terminal device 200A, while the camera device 210A transmits an image capturing the process from pressing the nail until its release, to the biological information estimating apparatus 100C. The color information acquisition device 120C acquires the color information from the captured image, and the biological information estimation unit 130C estimates biological information from the action information and color information.

As described, according to the biological information estimation apparatus 100C provided in the second embodiment, it is possible to estimate the patient's biological information based on the captured image of the patient, the acquired action information, and the corrected color information.

### (Third Embodiment)

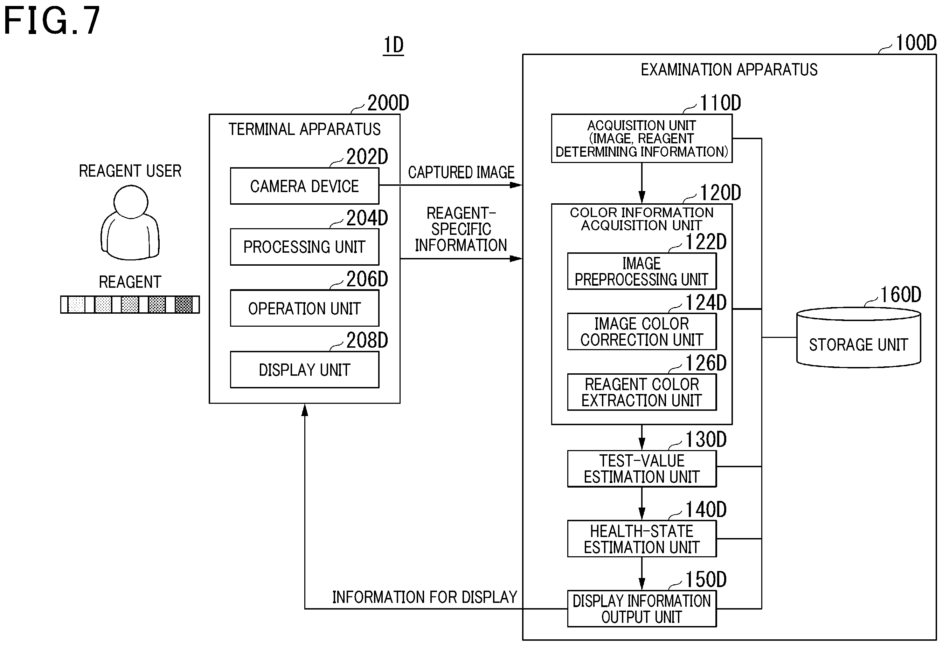

Fig. 7 is a block diagram exemplifying the configuration of an examination system 1D employed by a third embodiment. It is noted that the configuration elements described in the biological information estimation systems according to the foregoing embodiments can also be applied to the examination system 1D described in the explanation of the third embodiment.

In the examination system 1D, by way of example, the captured images of a reagent and reagent-specific information are transmitted to an examination apparatus 100D which is disposed remotely from a reagent user. The examination apparatus 100D acquires color information from the captured images and estimates test values with higher accuracy. The reagent employed in the present embodiment may be, for example, a urine test strip impregnated with the patient's urine, but is not limited thereto, and any known reagent where test values can be read based on the color is suitable.

The examination system 1D includes, for example, an examination apparatus 100D and a terminal device 200B. The examination apparatus 100D and the terminal device 200D are connected, for example, via a communication network. The examination apparatus 100D and terminal device 200D each have a communication interface, such as a NIC (Network Interface Card) or a wireless communication module, for connecting to a network such as the Internet. The communication network may include, for example, a general-purpose network such as the Internet, and private networks such as Local 5G or Wi-Fi (registered trademark).

The terminal device 200D is an information processing device, such as a smartphone or personal computer, used by the reagent user. The terminal device 200D is provided with, for example, a camera device 202D, processing unit 204D, an operation unit 206D, and a display unit 208D. The camera device 202D operates based on operations received by the terminal device 200D and generates images capturing the reagent. The processing unit 204D is a processor that executes application software to perform various processing and control tasks. The operation unit 206D is an operation interface such as a touch panel or buttons. The display unit 208D is a liquid crystal display or similar device. It is noted that in the present embodiment, the terminal device 200D and the examination apparatus 100D are separate units, although this is not a limitation. Alternatively, the functions possessed by the examination apparatus 100D may be incorporated into the terminal device 200D.

The processing unit 204D may read a three-dimensional code or the like described on the reagent using the camera device 202D and identify reagent-specific information in terms of the reagent manufacturer or other necessary information, through image recognition processing. As another example, the processing unit 204D may generate the reagent-specific information in response to receiving operations inputting information about the reagent via the operation unit 206D. Moreover, if the processing unit 204D is configured to execute dedicated application software corresponding to the reagent, it may execute the application software and, upon acquisition of captured images, transmit the images and reagent-specific information to the examination apparatus 100D. Furthermore, the processing unit 204D may recognize AR markers, registration marks, etc., and generate the reagent-specific information. Additionally, the processing unit 204D may predict a degree of paper degradation based on the captured image and check whether the reagent has expired.

The camera device 202D may be a camera built in a smartphone, a single-lens reflex camera, a webcam, etc. The terminal device 200D may transmit information indicating the camera type along with the captured images.

The captured images may be still images of the reagent or moving images of the reagent. The captured images may be a plurality of images generated by interval imaging at intervals of seconds, for example, to observe the reagent's reaction. The captured images may be a plurality of images captured by varying the subject distance, flash presence, angle, etc.

The captured images may be images captured with fixed shooting conditions (lighting, field of view) according to the case.

The captured images may be images capturing the reagent along with reference objects such as a color chart or white plate for color correction. The captured images may include both an image containing the reagent and an image capturing the reference object.

The captured images may include depth information measured using a depth sensor or similar device.

The inspection device 100D is an information processing device that communicates with other devices and performs various processes. The inspection device 100D includes, by way of example, an acquisition unit 110D, a color information acquisition device 120D, a test value estimation unit 130D, a health condition estimation unit 140D, a display information output unit 150D, and a storage unit 160D. The acquisition unit 110D, color information acquisition device 120D, test value estimation unit 130D, health status estimation unit 140D, and display information output unit 150D are functional units realized by a processor, such as a CPU (Central Processing Unit), executing programs stored in a program memory. The storage unit 160D is configured by any combination of storage media such as an HDD (Hard Disk Drive) or an SSD (Solid State Drive).

The acquisition unit 110D functions as an image acquisition unit that acquires an image which images (captures) the reagent, and as a reagent-specific information acquisition unit that acquires reagent-specific information for determining which reagent it is.

The color information acquisition device 120D corrects the color information included in the captured image and acquires the corrected color information. The color information is information indicating the color of the reagent for estimating test values. The color information may be RGB values reproduced in a specific color space, such as RGB values with white balance correction applied or sRGB values. The color information may be spectral reflectance, XYZ values in the XYZ color space, L*a*b* values in the L*a*b* color space, redness index, melanin index, absorbance, spectral data at specific wavelengths, representative values for specific regions of the subject, or time-series information showing temporal changes in images captured via video or interval photography with pixel-by-pixel values. The color information may also include two or more of these types of information. The color information may be a single value, such as the average value of the extracted body portion, or the color information may be image data of a small region containing the extracted body portion. For example, the image data may be composed of a two-dimensional distribution of color information.

The color information acquisition device 120D includes, by way of example, an image preprocessing unit 122D, an image-color correction unit 124D, and a reagent color extraction unit 126D.

The image preprocessing unit 122D performs preprocessing on captured images. The preprocessing precedes the color correction, and may include shading (illuminance unevenness) correction, specular reflection correction, and/or white balance estimation. The preprocessing may also include processing to facilitate reagent color extraction (such as trapezoidal correction, or processing to identify a reagent portion on the image and perform rectangular cropping therefrom).

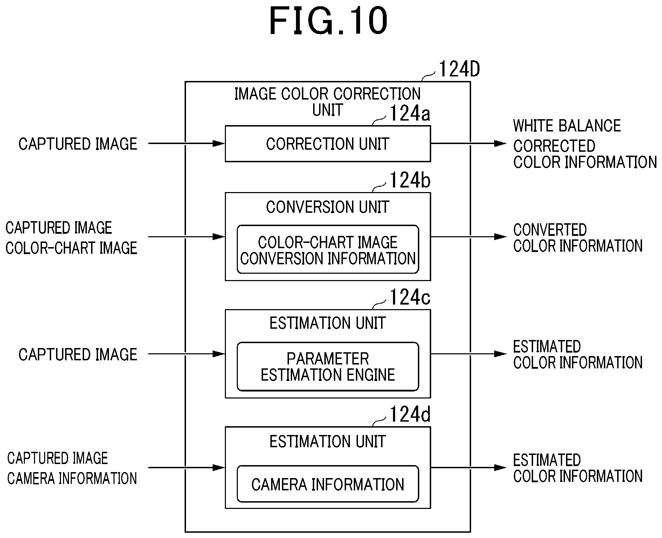

The image color correction unit 124D acquires color-corrected image information with the accurate color of the reagent, by correcting the color information included in the preprocessed captured images. The color correction will be explained with reference to Fig. 10.

The reagent color extraction unit 126D extracts the color of the reagent based on the corrected color information. If the reagent has multiple test areas as shown in Fig. 7, the color information is extracted for each area. The reagent color extraction unit 126D handles reagents with varying reaction times and potentially different recommended test times. In such a case, the reagent color extraction unit 126D extracts color information from the captured images taken at each recommended inspection time among the multiple images captured (imaged) at intervals. For videos, the color information is extracted from the frame at the recommended time. The reagent color extraction unit 126D may detect a reagent pattern (precipitation) instead of the color. The reagent color extraction unit 126D may determine reagents based on the extracted color.

The test values estimation unit 130D estimates the test values of the reagent based on the reagent-specific information and the color information. If the reagent has multiple test areas as described, the test values estimation unit 130D estimates the test value for each test area. The test value estimation unit 130D determines a test value based on the color information extracted for each test area by the reagent color extraction unit 126D and the distance to the representative color for each test item. The test value estimation unit 130D may estimate test values using methods such as multiple regression analysis, polynomial approximation, or machine learning (CNN, SVM, etc.).

The test values estimation unit 130D may input the images acquired by the acquisition unit 110D and the reagent-specific information into the machine learning engine that has learned the images of the reagent, the reagent-specific information, and the test values. In response to this input, the test values estimation unit 130D may output the test values, based on the output from the machine learning engine. Parameters for implementing the machine learning engine may be stored in the storage unit 160D, in which the machine learning engine has been trained for each reagent. During the examination, the test value estimation unit 130D may read a trained model corresponding to reagent-specific information from the storage unit 160D and input the color information into the trained model. Accordingly, the test value estimation unit 130D may estimate test values based on the output of the trained model.

The health-state estimation unit 140D estimates the health state of the reagent user based on the test values estimated by the test values estimation unit 130D. The health-state estimation unit 140D may estimate a disease name that functions as an indicator representing the health state. The information for display output unit 150D outputs to the terminal device 200D the test values estimated by the test values estimation unit 130D and the information for display for presenting the health state estimated by the health-state estimation unit 140D. The storage unit 160D stores information processed in the acquisition unit 110D, color information acquisition device 120D, test values estimation unit 130D, health-state estimation unit 140D, and information for display output unit 150D.

Incidentally it is noted that the reagent-specific information need only be information that determines the reagent, and, for example, the reagent-specific information may be information that determines the type of the reagent. In this case, the test value estimation unit 130D can estimate a reagent test value based on the reagent type and color information.

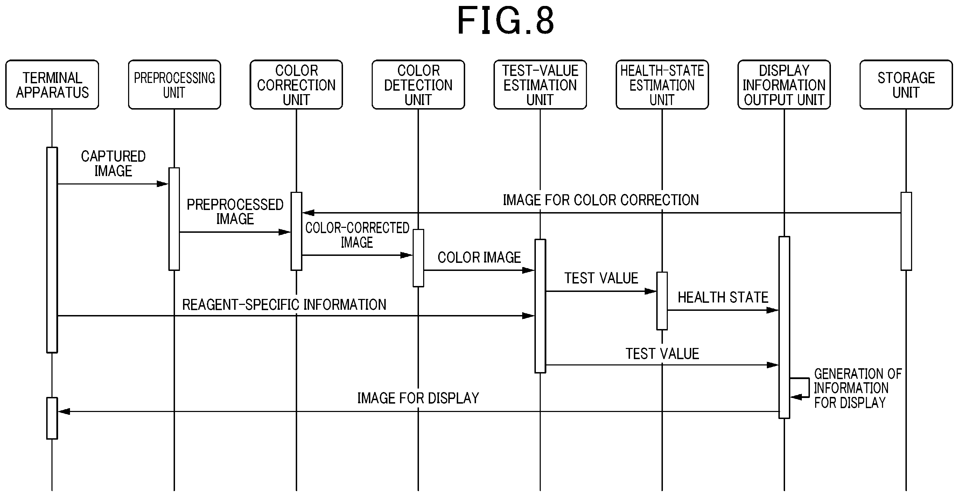

Fig. 8 is a sequence diagram showing an example of the processing steps performed by the examination system 1D according to the third embodiment.

First, the terminal device 200D images a reagent user using the camera device 202D, transmits the captured image to the examination apparatus 100D, and transmits the reagent-specific information to the examination apparatus 100D. The acquisition unit 110D acquires the captured image, while the image preprocessing unit 122D performs preprocessing on the captured image and outputs the preprocessed image to the image-color correction unit 124D. The image-color correction unit 124D acquires the information necessary for correction from the storage unit 160D and corrects the color information of the preprocessed image. The image-color correction unit 124D outputs the color-corrected image to the reagent color extraction unit 126D. The reagent color extraction unit 126D extracts the color of the reagent from the color-corrected image and outputs the extracted information about the color to the test value estimation unit 130D. The health-state estimation unit 140D estimates a health state based on the test values. The display information output unit 150D generates information for display including the test value and the health state and transmits the generated information to the terminal device 200D. Incidentally, the test value estimation unit 130D may output information about an error result from the information for display output unit 150D to the terminal device 200D in cases such as failure to detect the reagent color or failure to perform the health state examination by the health-state estimation unit 140D. The display information output unit 150D may generate and transmit, along with the error result, the display information to the terminal device 200D prompting the user to perform the re-imaging.

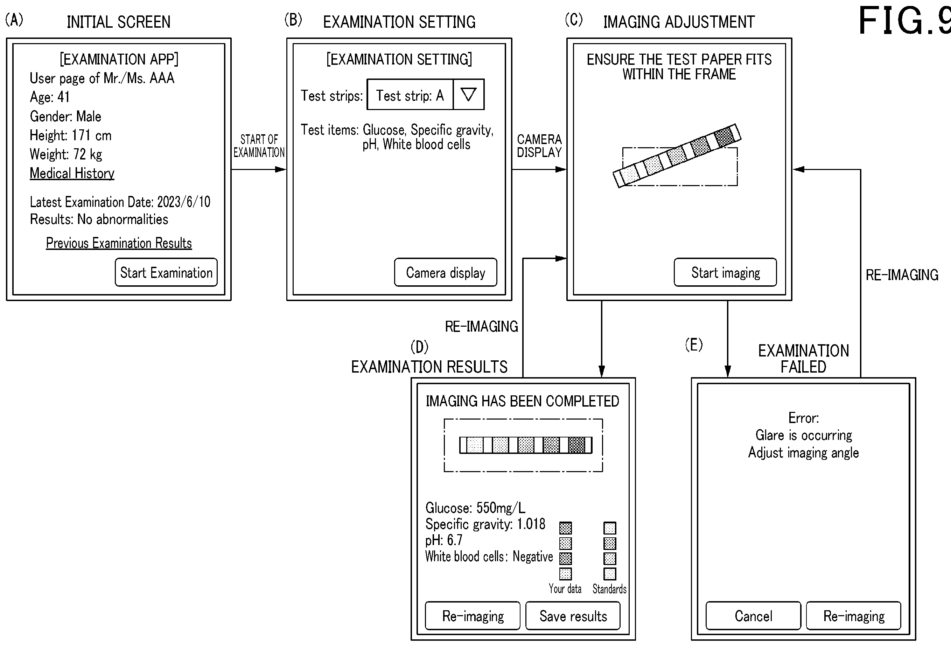

Fig. 9 is an example showing a display screen of the terminal device 200D provided in the third embodiment.

The terminal device 200D first displays the initial screen (user page) shown in part (A) of Fig. 9 by launching the application software. Then, the terminal device 200D displays a test settings screen shown in part (B) of Fig. 9 in response to detecting that the "Start exam." button has been selected via the operation unit 206D.

The test settings screen shown in part (B) of Fig. 9 allows the user to select a reagent from a dropdown list and includes test items corresponding to each of the reagents. When the reagent is selected, the terminal device 200D displays an imaging adjustment screen shown in part (C) of Fig. 9 in response to receiving an operation selecting the camera display button. Then, the terminal device 200D generates reagent-specific information corresponding to the selected reagent.

On the imaging adjustment screen shown in part (C) of Fig. 9, for example, a message stating "Ensure the test paper fits within the frame" and a guide frame are displayed on a camera image in a superimposed manner such that the message and guide frame are superimposed on the camera image. When the terminal device 200D detects that the examination start button has been selected while the imaging adjustment screen is displayed, the terminal device 200D captures the camera image within the frame as a captured image and transmits both the captured image and the reagent-specific information to the examination apparatus 100D.

The terminal device 200D receives the information for display from the examination apparatus 100D and displays either an examination result screen shown in part (D) of Fig. 9 or an examination failure screen shown in part (E) of Fig. 9 based on the received information for display.

The part (D) of Fig. 9 shows the examination result screen, which includes the reagent image serving as the imaging result, test results (numerical values, health status), reference images, a re-imaging button, and a result save button. This allows the reagent user to view the test values and the health status. The terminal device 200D displays the imaging adjustment screen (part (C) of Fig. 9) when the re-imaging button is selected. The terminal device 200D displays the initial screen (part (A) of Fig. 9) when the result save button is selected. The terminal device 200D may send the examination results, etc., to other application software or server devices when the save result button is selected, thereby providing services linked to the examination results. The examination failure screen (part (E) of Fig. 9) includes information indicating errors, a cancel button, and a re-imaging button. This allows the terminal device 200D to prompt re-imaging the reagent.

Fig. 10 is an example diagram showing the configuration of the image-color correction unit 124D according to the third embodiment.

The color information acquisition device 120D includes an image-color correction unit 124D. This image-color correction unit 124D may include at least one of a correction unit 124a, a conversion unit 124b, an estimation unit 124c, and an estimation unit 124d. The correction unit 124a, conversion unit 124b, estimation unit 124c, and estimation unit 124d are functional units functionally realized, for example, by a processor such as a CPU executing programs stored in a program memory.

The correction unit 124a estimates an illumination color of the space where the reagent was imaged, based on the captured image, and corrects the white balance of the RGB information included in the captured image. It is desirable for the background of the captured image to include an auxiliary background such as white. A message may be displayed prompting the user to capture the image in an environment containing a white background when using the camera device 202D. The correction unit 124a outputs the captured image containing the color information with corrected white balance, to the reagent color extraction unit 126. The correction unit 124a may modify the correction process according to the type of reagent.

The acquisition unit 110D acquires a color chart image captured from a color chart and an image captured from a reagent. The conversion unit 124b generates conversion information that converts the color information included in the image captured from the reagent using the color chart image, and converts the color information of the image based on the conversion information. The color chart may be, for example, a plate-shaped object with color samples arranged thereon. Alternatively, instead of the color chart, an image of the reagents may be acquired before impregnation and used as a color chart or reference. The conversion unit 124b generates a color conversion matrix (conversion information) based on the relationship between the colors indicated by the color chart and the colors of the color chart contained in the captured image. The conversion unit 124b generates the color conversion matrix using the color chart, for example, by performing multiple regression analysis. The conversion unit 124b can also convert the captured image of the reagents to the colors indicated by the color chart by converting the captured image based on the conversion information. The conversion unit 124b outputs the captured image with the converted color information to the reagent color extraction unit 126D. The conversion unit 124b may generate the conversion information for each reagent type.

The estimation unit 124c estimates color information based on output information from a machine learning engine that estimates parameters affecting the color of captured images when imaging a reagent. The parameters affecting the color of captured reagent images include, for example, spectral reflectance and absorbance. The machine learning engine is a parameter estimation engine trained using captured images and parameters (such as spectral reflectance or absorbance) as training data. The parameter estimation engine estimates parameters in response to the input of a captured image. The estimation unit 124c estimates the color information of the captured image using the parameters estimated by the parameter estimation engine.

By way of example, the estimation unit 124c may generate other parameters using the absorbance estimated by the parameter estimation engine. The estimation unit 124c estimates the color information of the captured image using other parameters generated using the parameters estimated by the parameter estimation engine.

The estimation unit 124D estimates the exact color information based on the captured image acquired by acquisition unit 110D and information about the color characteristics of the camera device used for the imaging, such as spectral sensitivity. The terminal device 200 transmits camera information related to the color information, such as the type and specifications (e.g., sensitivity) of the camera device 202D, along with the captured images to the examination apparatus 100D. The estimation unit 124d may estimate the reagent user's color information based on the camera information contained within the captured image.

As described, according to the examination system 1D according to the embodiment, an image capturing a reagent user is acquired, the color information contained in the captured image is corrected, and the biological information of the reagent user is estimated based on the corrected color information. In this way, the color information is corrected even for various captured images, that is, regardless of the types of camera devices 202D which are used to capture the image or factors of environments surrounding reagent users. This enables the acquisition of color information from the captured image and the estimation of biological information with higher accuracy.

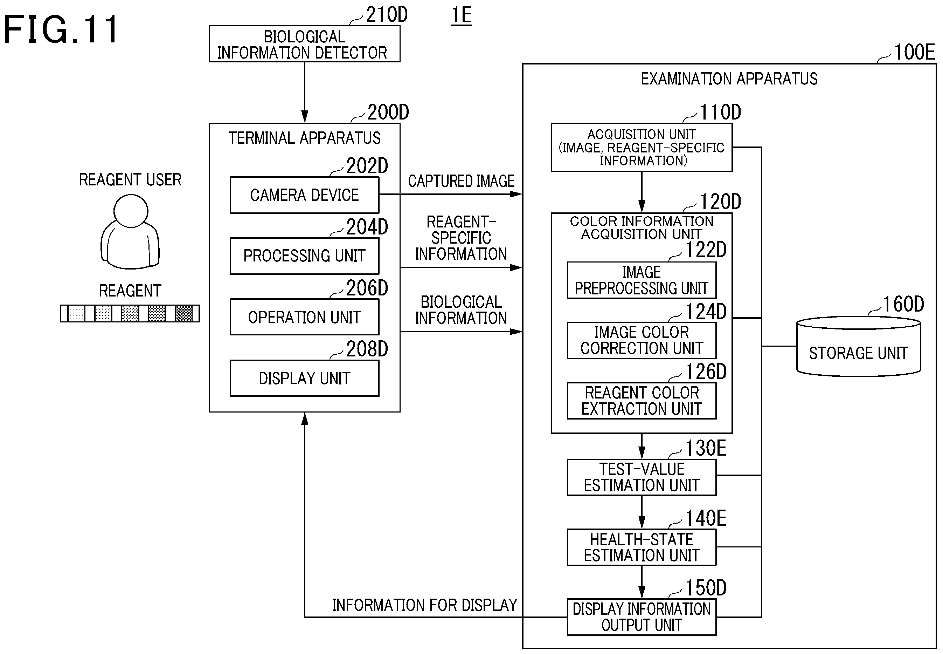

Fig. 11 is a block diagram showing an example of the examination system 1E according to the third embodiment.

The examination system 1E includes a biological information detector 210D that acquires biological detection information detected from a reagent user. The biological information detector 210D may be provided as a device, such as a smartwatch, that measures the biological information of the reagent user. Alternatively, this biological information detector 210D may also be provided as a device that accepts operations from the reagent user or the like to input biological information thereto. The biological information detected by the biological information detector 210D is biological information that assists the health-state estimation unit 140E in estimating the health state.

The biological information detected by the biological information detector 210D may include personal information such as gender, height, weight, age, and medical history; detection values from the biometric sensor such as body temperature, oxygen saturation, pulse rate, blood pressure, and electrocardiogram; and environmental information detected by environmental sensors such as temperature, humidity, and time. Among pieces of the information detected by the terminal device 200D, environmental information where the reagent color may change may be transmitted to the examination apparatus 100E to assist the test value estimation unit 130E in estimating test values.

The biological information detector 210D may be communicably connected to a server device such that biological information detector 210D is able to acquire blood test results (hemoglobin), urine test results, various test results (melanin, etc.), medical history, etc., from the server device. In addition, the biological information detector 210D may estimate biological information from the captured images obtained by the camera device 202D or by other means through internal processing. Alternatively, the biological information detector 210D may be configured to estimate, for example, burns, injuries, melanin, etc., from the captured images.

The test value estimation unit 130E can estimate test values based on environmental information indicating the environment where the reagent was used, the color information, and the reagent-specific information. The health-state estimation unit 140E can also estimate health statuses based on the biological information acquired from the biological information detector 210D and the test values.

As described, according to the examination apparatus 100E according to the embodiment, in addition to the captured image of the reagent, biological detection information acquired by the biological information detector 210D can be obtained. Based on the corrected color information and the acquired biological information, the health status can be estimated. In addition, the examination apparatus 100E can estimate test values with higher accuracy, based on environmental information indicating the environment where the reagent was used, color information, and reagent-specific information.

The terminal device and biological information estimating apparatus described in the foregoing embodiments may be implemented on a computer. In such a case, the described functionality may be realized by recording programs for implementing the described functionality onto a computer-readable recording medium, loading the program recorded on this medium into a computer system, and executing the programs. In the present disclosure, the term "computer system" includes hardware such as an operating system and peripheral devices. The term "computer-readable recording medium" refers to storage devices such as portable media (e.g., floppy disks, magneto-optical disks, ROM, CD-ROM) or internal hard disks within a computer system. Furthermore, the "computer-readable recording media" may also include: - Media that dynamically hold a program for a short time, such as communication lines when transmitting programs via networks like the Internet or communication lines like telephone lines; and - Media that hold programs for a certain period, such as volatile memory within the computer system acting as the server or client in such cases. Moreover, the foregoing programs may be ones that implement only part of the previously described functions. Such programs may also be ones that implement the previously described functions in combination with programs already recorded in the computer system. Such programs may also be ones implemented using programmable logic devices such as FPGAs (Field Programmable Gate Arrays).

For describing some of the configurations of the biological information estimating apparatus in the embodiments, the term "unit" is used. However, the term "unit (functionally identical to "Bu" in Japanese)" may be read as other terms representing the technical configuration, structure, function, or action of the biological information estimating apparatus, such as circuit, module, component, or other identical terms.

Moreover, the terms such as "Bu (functionally meaning "unit" in Japanese)," "circuit," "unit," "module," "component," and the like may also refer to hardware configured to execute software objects or software routines, and/or modules or components that may be stored and/or executed by general-purpose computer system hardware (e.g., computer-readable media, processor devices, etc.).

The embodiments and variations described above are merely illustrative examples and are not intended to limit the scope of the present invention. For example, any one of the embodiments or variations, or a portion of any one of the embodiments or variations, may be combined with one or more other embodiments or one or more other variations to realize another aspect of the present invention.

### (Appendix)

The embodiment modes can be developed as follows:
(1-1) There is provided a biological information estimation apparatus equipped with an image acquisition unit for acquiring an image capturing a living body;
   a color information acquisition device for correcting color information included in the captured image and acquiring the corrected color information; and
   a biological information estimation unit for estimating biological information of the living body based on the color information.
(1-2) In the biological information estimation apparatus described in (1-1), the color information acquisition device estimates illumination color of the space in which the living body was captured based on the captured image, and corrects a white balance of RGB information included in the captured image.
(1-3) In the biological information estimation apparatus described in (1-2), the image acquisition unit acquires a color chart image capturing the color chart and an image capturing the living body, and
   the color information acquisition device generates conversion information used for converting the color information included in the image capturing the living body, using the color chart image, and then converts the color information included in the captured image based on the conversion information.
(1-4) In the biological information estimation apparatus described in (1-1), the color information acquisition device estimates the color information based on output information from a machine learning engine that estimates parameters affecting the color of captured images when imaging the living body.
(1-5) In the biological information estimation apparatus described in (1-1), the color information acquisition device estimates the color information of the living body based on camera information included in the captured image.
(1-6) In the biological information estimating apparatus described in (1-1), the color information acquisition device estimates the color information, based on body portion information acquired by the image acquisition unit or output information from the machine learning engine that recognizes a body portion of the living body.
(1-7) In the biological information estimation apparatus described in (1-1), the apparatus is provided with a biological information detector acquiring biological detection information detected from the living body, and the biological information estimation unit estimates the biological information based on the biological detection information acquired by the biological information detector and the color information.
(1-8) In the biological information estimation apparatus described in (1-1), the apparatus is provided with an action information acquisition unit acquiring action information of the living body provided when imaging the living body, wherein color information acquisition device acquires the color information of the living body based on the obtained image and the action information.
(1-9) In the biological information estimation apparatus described in (1-1), the image acquisition unit acquires an image capturing a patient which serves as the living body,
   the color information acquisition device corrects the color information of the patient, and
   the biological information estimation unit estimates the biological information of the patient.
(1-10) There is provided a biological information estimation method, the method including steps of:
   acquiring an image capturing a living body, which is performed by a biological information estimating apparatus;
   correcting color information included in the image and acquiring the corrected color information, which is performed by the biological information estimating apparatus; and
   estimating biological information of the living body based on the color information, which is performed by the biological information estimating apparatus.
(1-11) A program executed by a computer equipped in an information processing device, providing:
   a step of acquiring an image in which a living body is imaged;
   a step of correcting color information included in the image and acquiring the corrected color information; and
   a step of estimating biological information of the living body based on the color information.
(2-1) There is provided with an examination apparatus equipped with:
   an image acquisition unit for acquiring an image in which a reagent is imaged;
   a reagent-specific information acquisition unit for acquiring reagent-specific information to determine the reagent;
   a color information acquisition device for correcting color information included in the captured image and acquiring the corrected color information; and
   a test value estimation unit for estimating a test value of the reagent based on the reagent-specific information and the color information.
(2-2) In the examination apparatus described in (2-1),
   the reagent-specific information is provided as information for determining a type of the reagent, and
   the test value estimation unit estimates the test value of the reagent based on the type of the reagent and the color information.
(2-3) In the examination apparatus described in (2-1), the color information acquisition device estimates an illumination color in a space in which the reagent is imaged, based on the image capturing the reagent, and corrects a white balance of RGB information included in the image.
(2-4) In the examination apparatus described in (2-1),
   the image acquisition unit acquires a color chart image capturing a color chart and the image capturing the reagent, and
   the color information acquisition device generates, by using the color chart image, conversion information for conversion of the color information from the image of the reagent, and the color information included in the image of the reagent based on the generated conversion information.
(2-5) In the examination apparatus described in (2-1), the color information acquisition device estimates the color information based on information outputted from a machine learning engine which estimates parameters affecting on colors of the image of the reagent during imaging thereof.
(2-6) In the examination apparatus described in (2-1), the color information acquisition device estimates the color information of the reagent based on camera information included in the image.
(2-7) In the examination apparatus described in (2-1), the test value estimation unit applies, as input, the image acquired by the image acquisition unit and the reagent-specific information acquired by the reagent-specific information acquisition unit to a machine learning engine trained with the image of reagent, the reagent-specific information, and the test value, and makes the machine learning engine output the test value.
(2-8) In the examination apparatus described in (2-1), the apparatus is further provided with a health-state estimation unit estimating a health state of a user who uses the reagent, based on the test value estimated by the test value estimation unit.
(2-9) In the examination apparatus described in (2-8), the apparatus is further provided with a biological information detector which acquires biological information detected from the user of the reagent, and the health-state estimation unit estimate the health state, based on the biological information acquired by the biological information detector, the test value estimated by the test value estimation unit, and the reagent-specific information.
(2-10) In the examination apparatus described in (2-1), the test value estimation unit estimates the test value based on environmental information representing an environment in which the reagent is used, the color information, and the reagent-specific information.
(2-11) There is provided an examination method including:
   a step of acquiring an image in which a reagent is imaged, the acquiring step being performed by an examination apparatus;
   a step of acquiring reagent-specific information for determining the reagent, the acquiring step being performed by the examination apparatus;
   a step of correcting color information included in the image and acquiring the corrected color information, the correcting step being performed by the examination apparatus; and
   a step of estimating a test value of the reagent based on the reagent-specific information and the color information, the estimating step being performed by the examination apparatus.
(2-12) There is provided a program which enables a computer installed in an information processing device to execute steps of:
   acquiring an image in which a reagent is imaged;
   acquiring reagent-specific information for determining the reagent;
   correcting color information included in the image and acquiring the corrected color information; and
   estimating a test value of the reagent based on the reagent-specific information and the color information.

### [Partial Reference Signs List]

1, 1A, 1B, 1C Biological information estimation system
1D, 1E Examination system
100, 100A, 100B, 100C Biological information estimation apparatus
100D, 100E Examination apparatus
110, 110A Image acquisition unit
110D Acquisition unit
120 Preprocessing unit
120A, 120C, 120D Color information acquisition device
121A, 124a Correction unit
122A, 124b Conversion unit
122D Image preprocessing unit
123A, 124A, 124c, 124d, 125A Estimation unit
124D Image-color correction unit
126D Reagent color extraction unit
130 Extraction unit
130A, 130B, 130C Biological information estimation unit
130D, 130E Test-value estimation unit
140 Estimation unit
140D, 140E Health-state estimation unit
150 Output unit
150D Display information output unit
160, 160D storage unit
200, 200A, 200B, 200D Terminal device
202D Camera device
204, 204D Processing unit
206D Operation unit
208D Display unit
210, 210A Camera device
210D, 220A Biological information detector
220 Input unit
230 Display unit
230A Action information acquisition unit

## Claims

1. A biological information estimation apparatus comprising:
an image acquisition unit that acquires an image capturing a subject;
a preprocessing unit that performs a predetermined preprocessing on the captured image;
an extraction unit that extracts color information of the subject from the captured image preprocessed by the preprocessing unit;
an estimation unit that estimates a biological information value based on the color information and subject information indicating information about the subject, the biological information value being a value indicating biological information; and
an output unit that outputs the biological information value.

2. The biological information estimation apparatus according to claim 1, wherein the preprocessing to be performed in the preprocessing unit includes a color correction process, the color correction process correcting a color of the subject included in the captured image to approximate an actual color of the subject.

3. The biological information estimation apparatus according to claim 2, comprising a biological information determining unit that determines the biological information estimated by the estimation unit,
wherein the color correction performed by the preprocessing unit is performed corresponding to the biological information determined by the biological information determining unit.

4. The biological information estimation apparatus according to claim 2, comprising a subject determining unit that determines the subject,
wherein
the preprocessing unit performs the color correction corresponding to the subject determined by the subject determining unit, and
the estimation unit estimates the biological information value based on the subject information corresponding to the subject determined by the subject determining unit.

5. The biological information estimation apparatus according to claim 4, wherein the preprocessing unit switches conversion information indicating a correspondence between color information of the captured image and a colorimetric value of the subject, based on a type of the subject determined by the subject determining unit

6. The biological information estimating apparatus according to claim 4, wherein
the preprocessing unit estimates a spectral reflectance by applying the captured image to a machine learning model trained using both color information of captured images specifically assigned to the subject and a spectral reflectance of actual subjects, and performs the color correction for the captured image, based on a colorimetric value of the subject which obtained from the estimated spectral reflectance.

7. The biological information estimation apparatus according to claim 2, wherein
the preprocessing unit extracts relevant information from the captured image, the relevant information being concerning capture of the image, and performs the color correction based on the extracted relevant information.

8. The biological information estimation apparatus according to claim 7, wherein
the preprocessing unit estimates an illumination color in a subject surrounding, the illumination color serving as the relevant information, and performs the color correction based on the estimated illumination color.

9. The biological information estimation apparatus according to claim 7, wherein
the preprocessing unit performs the color correction based on camera model information included in metadata of the captured image, the camera model information serving as the relevant information.

10. The biological information estimation apparatus according to claim 2, wherein
the preprocessing unit performs the color correction based on registration information received based on user operations.

11. The biological information estimation apparatus according to claim 10, wherein
the registration information is illumination color, and
the preprocessing unit performs the color correction based on the illumination color.

12. The biological information estimation apparatus according to claim 10, wherein
the registration information is terminal device information, and
the preprocessing unit performs the color correction based on camera model information corresponding to the terminal device information.

13. The biological information estimation apparatus according to claim 2, wherein
the subject information represents operations inducing temporal changes in the subject, and
the estimation unit estimates the biological information value based on changes in the color information corresponding to the temporal changes in the subject.

14. The biological information estimation apparatus according to claim 1, wherein
the subject information is biologically relevant information pertaining to an individual user, and
the estimation unit estimates the biological information value based on the color information and the biologically relevant information.

15. The biological information estimation apparatus according to claim 1, wherein
the subject information is biologically relevant information or environmental information, which pertains to an individual user, and
the estimation unit estimates the biological information value based on the color information and the biologically relevant information.

16. The biological information estimation apparatus according to claim 1, wherein
the image acquisition unit acquires an image capturing the subject and a color chart image capturing a color chart, and
the preprocessing unit generates conversion information for converting color information included in the captured image of the subject, using the color chart image, and corrects the color information included in the captured image based on the conversion information.

17. The biological information estimation apparatus according to claim 1, comprising a health-state estimation unit that estimates a health state based on the biological information value estimated by the estimation unit.

18. A biological information estimation method, which is performed by a computer, comprising steps of:
acquiring an image capturing a subject;
performing a predetermined preprocessing on the captured image;
extracting color information of the subject from the captured image preprocessed by the preprocessing unit;
estimating a biological information value based on the color information and subject information indicating information about the subject, the biological information value being a value indicating biological information; and
outputting the biological information value.

19. A biological information estimation program which enables a computer to perform steps of:
acquiring an image capturing a subject;
performing a predetermined preprocessing on the captured image;
extracting color information of the subject from the captured image preprocessed by the preprocessing unit;
estimating a biological information value based on the color information and subject information indicating information about the subject, the biological information value being a value indicating biological information; and
outputting the biological information value.
